# EUROPEAN PATENT APPLICATION

(11) **EP 4 595 985 A1**
(43) Date of publication of application: **06.08.2025**
(21) Application number: 24382095.8
(22) Date of filing: 31.01.2024
(51) Int. Cl.: A61K 51/12, A61P 35/00

(54) **223RA CORE-DOPED IRON OXIDE NANOPARTICLES, PREPARATION METHOD AND USE THEREOF**

(71) Applicant: Consejo Superior De Investigaciones Científicas, 28006 Madrid (ES); King's College London, London WC2R 2LS (GB)
(72) Inventor: Herraiz Pérez, Aitor, Madrid (ES); Herranz Rabanal, Fernando, Madrid (ES); Pellico Sáez, Juan, Londres (GB); Torres Martin de Rosales, Rafael, Londres (GB)
(74) Representative: Pons IP

(57) **Abstract**

The present invention relates to nanoparticles comprising a core of iron oxide doped with ²²³Ra and a coating of an organic compound. The invention also relates to a method for their preparation and to the use thereof, particularly in both diagnosis and therapy.

## Description

The present invention relates to iron oxide nanoparticles (IONPs) doped with ²²³Ra and methods of synthesizing the same. Also, the invention relates to their use in both diagnosis and therapy.

Therefore, the present invention belongs to the field of medicine.

### BACKGROUND ART

Cancer is one of the leading diseases worldwide, with more than 37.5 million diagnosed cases in the last few years and high levels of incidence, prevalence, and mortality. Nowadays, several cancer treatments are available for improving the life expectancy of cancer patients, such as surgical intervention, radiation therapy and chemotherapy. However, treatment inefficiency or significant toxicity remain as the main challenges of current available oncologic therapies. Radiotherapy, which is a therapy wherein ionizing radiation is used to control or kill cells of undesirable growth, has been used for cancer treatment. It is based in the use of a radiation source (e.g.: a radioactive source, such as radiometals) to generate a beam of radiation.

The use of radiometals (RM) with imaging and/or therapeutic properties is becoming more and more widespread for application in different pathologies. A common aspect of these RM is the need to complex them, to bind them to some kind of organic compound that binds as strongly as possible to the radiometal (these are called chelating agents or chelators). A characteristic of these chelating agents is that they bind stably to the RM, so that that they do not "release" the RM once injected in vivo. Furthermore, ideally, the binding of the RM and chelator should be easy, fast and should allow for subsequent binding of biomolecules so that their accumulation in the pathology of interest can be targeted. One of the most widely used chelators is DOTA (dodecane tetraacetic acid), which has been used to complex radioisotopes such as ⁶⁴Cu, ⁶⁸Ga, ¹¹¹In and some others RM (Baranyai, Z., Tircsó, G., & Rösch, F. Eur. J. Inorg. Chem. 2020 (1), 36-56).

The problems associated to traditional chelators span from low in vivo stability, leading to RM transmetallation to limited number of applications due to their low molecular weight.

The radioactive isotope ²²³Ra is used in radiotherapy for castration resistant prostate cancer with bone metastasis (CRPC), being one of the RM with better properties as radiopharmaceutical. However, the problem is that ²²³Ra chemistry hinders its stable incorporation into traditional chelators. Because of this, ²²³Ra is only used in CRPC since the isotope itself has a high affinity towards bones but, until now, it has been difficult to bind it stably to biomolecules that could target its radiation to other types of tumours.

Radiolabelled iron oxide nanoparticles have been used in imaging and therapy in the state of the art. For example, next documents disclose this type of nanoparticles for imaging or therapy purposes: Yaser Hadi Gholami et. al. Scientific Reports (2019) 9:14346, Olga Mokhodoeva et al. J Nanopart Res (2016) 18:301, document WO 2015/054487 A1 and US 2006024235. However, these nanoparticles present a major inconvenient: the isotope ²²³Ra is adsorbed on the surface of the nanomaterial, this can easily lead to the leakage of the isotope *in vivo,* as it has been often demonstrated.

Then, with the aim of solving the above-mentioned inconveniences, the present invention provides new nanoparticles doped with ²²³Ra useful in both diagnosis and therapy.

### SUMMARY OF THE INVENTION

The present invention presents an innovative and previously unexplored approach for the design of novel theranostic agents, more specifically, radiotheranostic agents. In particular, the inventors of the present invention have found that ²²³Ra is incorporated in the core of iron oxide nanomaterials with a very large yield (90%) and, more importantly, the stability of the labelling in physiological conditions is close to 100 % avoiding the problems of *in vivo* leakage, which is typical of other nanoparticles known in the prior art. Furthermore, the nanoparticles of the present invention can provide signal in imaging techniques like magnetic resonance imaging.

The term "radiotheranostic" refers to a compound enabling radiotherapy as well as diagnostic.

Then, a first aspect of the present invention relates to nanoparticles (NPs) (hereinafter, the NPs of the present invention) that comprise or consist of:
- an iron oxide core doped with radioisotope ²²³Ra (the ²²³Ra is within the crystalline structure of the iron oxide and being part of said structure, occupying vacancies corresponding to Fe²⁺ or Fe³⁺),
- a coating layer of an organic compound selected from: sodium citrate, dextran, carboxydextran, poly(vinyl alcohol) (PVA), 2,3-dimercaptosuccinic acid, 1-aminoglucose and glutamate.

The coating is bonded to the core by any type of an interaction, such as, but not limited to, covalent bonds, ionic bonds, dipole-dipole interactions, hydrogen bonds and the like.

In a preferred embodiment, the iron oxide is selected from FeO, Fe₃O₄, Fe₄O₅, Fe₂O₃ or FeOs. In a more preferred embodiment, the iron oxide is selected from maghemite (Fe₂O₃) or magnetite (Fe₃O₄).

The NPs of the present invention are extremely small. Preferably, they present an average diameter of the iron oxide core between 2 and 6 nm, as measured by Transmission Electron Microscopy (TEM), and/or a hydrodynamic average diameter of the nanoparticle between 10 and 20 nm, as measured by dynamic light scattering measurement (DLS) in phosphate buffered saline at pH 7.2. Then, the layer of organic material is usually around 8-14 nm and this point is important since not many nanoparticles have such a large organic coating which affects in their stability.

In a preferred embodiment, the NPs of the present invention comprises a coating of sodium citrate.

In a preferred embodiment, the specific activity of ²²³Ra isotope ranges from 0.5 GBq/mmol Fe to 8 GBq/mmol Fe measured in activimeter (CRC-PC 55t Smart Chamber from Canpintec).

Another aspect of the present invention refers to the method for preparing the NPs described in the first aspect of the present invention. The method comprises the following steps:
a) preparing a solution in water of a combination of: an iron salt a ²²³Ra salt, hydrazine hydrate and an organic compound selected from sodium citrate, dextran, carboxydextran, poly(vinyl alcohol) (PVA), 2,3-dimercaptosuccinic acid and 1-aminoglucose and glutamate ( hydrazine hydrate is used to initiate the reaction),
b) subjecting said mixture to a temperature between 90 and 120°C with microwave (MW) irradiation at 220-300W for 3-11 min,

In a preferred embodiment, the iron salt is selected from the group consisting of FeCl₃, Fe(SO₄), Fe₂(SC₄)₃, Fe(NO₃)₃, FeCOs, Fe(OH)₂, Fe(OH)₃ or any hydrate thereof. More preferably, the iron salt is iron (III) chloride hexahydrate.

In another preferred embodiment, ²²³Ra salt is ²²³RaCl₂.

In a preferred embodiment, the molar proportion iron salt:²²³Ra salt is 1:1 in the solution prepared in step a).

In a preferred embodiment, the iron salt or the ²²³Ra salt is in a concentration of 15-45 mM in the solution prepared in step a).

In a preferred embodiment, the hydrazine hydrate is used in excess (molar proportion iron salt: hydrazine hydrate higher than one).

In another preferred embodiment, the organic compound is sodium citrate.

In another preferred embodiment, step b) is carried out at 100°C for 10 min with MW irradiation.

In another preferred embodiment, the NPs obtained in step b) are purified. Preferably, they are purified by size-exclusion chromatography (SEC).

The present invention also refers to the NPs obtained for the above-described method.

Another aspect of the invention relates to a pharmaceutical composition comprising the nanoparticles of the present invention and at least one pharmaceutically acceptable vehicle or excipient.

The "vehicle" is preferably an inert substance. The function of the vehicle is to facilitate the incorporation of other compounds, to allow for better dosage and administration, or to give consistency and shape to the pharmaceutical composition. Thus, the vehicle is a substance which is used in the pharmaceutical composition to dilute any of the components of the pharmaceutical composition to a given volume or weight; or which, even without diluting said components, is capable of allowing better dosage and administration or giving consistency and shape to the medicament. When the form of presentation is liquid, the pharmaceutically acceptable vehicle is the diluent.

The term "excipient" refers to a substance that aids the absorption of any of the components of the composition of the present invention, stabilizes said components or aids in the preparation of the pharmaceutical composition in the sense of giving it consistency or providing flavors that make it more pleasant. Therefore, the term "excipient" is defined as that matter which, included in the galenic forms, is added to the active ingredients or their associations to enable their preparation and stability, modify their organoleptic properties or determine the physico-chemical properties of the pharmaceutical composition and its bioavailability. The "pharmaceutically acceptable" excipient must allow the activity of the compounds of the pharmaceutical composition, i.e. it must be compatible with these components.

In addition, as understood by the person skilled in the art, the excipient and vehicle must be pharmacologically acceptable, i.e., the excipient and vehicle must be permitted and evaluated so as not to cause harm to the organisms to which it is administered.

The pharmaceutical composition can be presented in any clinically permissible form of administration and in a therapeutically effective amount. In a preferred embedment, of the present invention, the pharmaceutical composition is in a form adapted for intravenous administration.

Another aspect of the present invention refers to the NPs defined in the first aspect of the present invention, or a pharmaceutical composition comprising thereof, for use as a medicament.

Another aspect of the present invention refers to the NPs defined in the first aspect, or a pharmaceutical composition comprising thereof, for use as radiotheranostic agents (both radiotherapy and diagnosis agents), that is, for use in the diagnosis and/or radiotherapy.

In a preferred embodiment, the present invention refers to the NPs defined in the first aspect for use in the detection and/or treatment of cancer.

In a preferred embodiment, said cancer is selected from glioblastomas, glioblastoma, hepatocarcinoma, pancreatic cancer and head-neck cancer.

In a preferred embodiment, the NPs are administrated intravenously for their use in diagnosis and/or therapy.

In a preferred embodiment, the diagnosis is carried by providing *in vivo* signals for positive contrast MRI (Magnetic Resonance Imaging).

The NPs described in the present invention present notable advantages with respect to radiolabelled iron oxide NPs that are known in the state of the art. These advantages are summarized below:
- Extremely small iron oxide nanoparticles with radioisotope ²²³Ra inside of the crystal structure of the core. This is a substantial difference from studies where different metals are mineralized on the surface of the iron oxide nanoparticle, as the exposure of ²²³Ra being inside of the crystal structure plays an important role in increasing *in vivo* stability and reducing free radium scattering by the organism. In the NPs of the prior art, isotope ²²³Ra is adsorbed on the surface of the nanomaterial, so that it can easily lead to the leakage of the isotope *in vivo.* The present invention avoids this problem.
- Thick organic layer providing colloidal stability in highly saline solutions and helping to the radiolabeling stability.
- Because of their small size, ²²³Ra-IONP not only are ideal candidates for radiotherapy in oncology but, at the same time, they provide positive contrast (bright signal) in Magnetic Resonance Imaging (MRI) so that they can be used as radiotheranostic agents.
- The NPs can be utilized for medical imaging during treatment, enabling the monitoring of treatment accumulation and progress.
- Due to the size of the Ra²⁺ cation (162 pm of crystal ionic radius), it was "surprising" how efficient the labelling was considering that the radium (²²³Ra) is occupying vacancies in the structure corresponding to Fe²⁺ (92 pm) or Fe³⁺ (78.5 pm). Unlike other radioisotopes, such as ⁶⁸Ga and ⁸⁹Zr, ²²³Ra has a larger size and low charge density so the effective doping of iron oxide nanoparticles with this radioisotope was unpredictable to the expert in the field, due its large size compared to iron. Therefore, such efficient doping, which has made it possible to obtain highly effective radiotheranostic agents, is an unexpected and surprising fact.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples are provided by way of illustration and are not intended to be limiting of the present invention.

### DESCRIPTION OF THE DRAWINGS

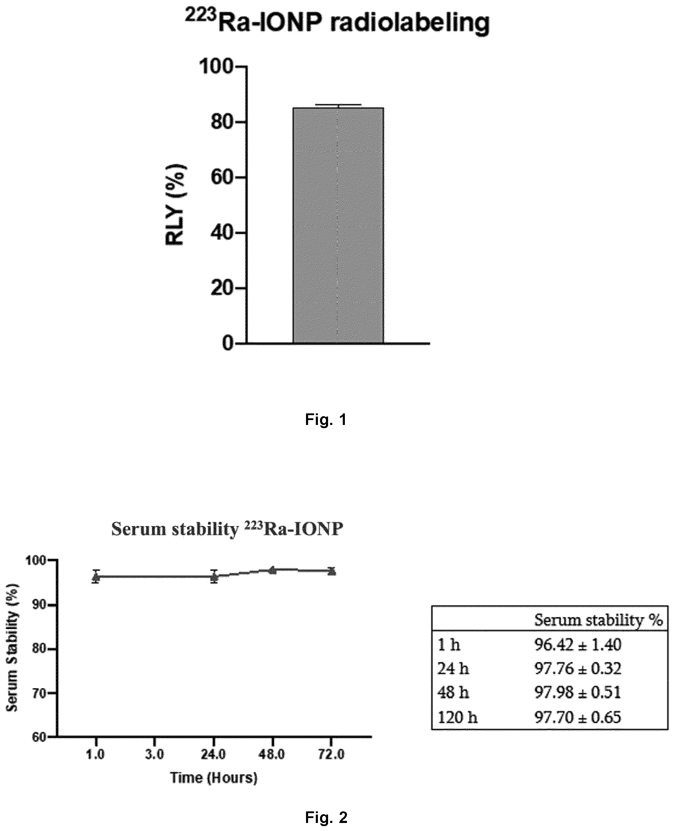

Fig. 1. Graph that shows the radiolabelling yield (RLY) in ²²³Ra-IONP radiolabelling according to example 1.

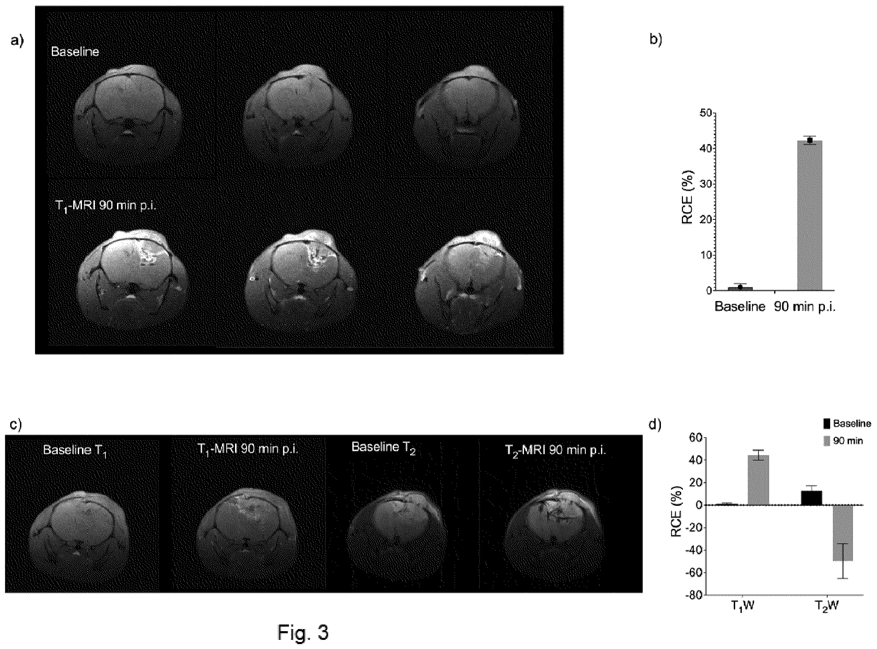

Fig. 2. Graph that shows the labelling stability after incubation in mouse serum. Results indicate that there is no leakage of ²²³Ra from the NPs. Fig. 3. Localisation of IONP in glioma mouse model by MRI. a) T₁-MRI of glioblastoma before (baseline) and after the administration of IONP; b) quantification of the signal measured in a) expressed as the relative contrast enhancement (RCE); c) confirmation of the localisation of the IONP in glioblastoma using both T₁-MRI and T₂-MRI; d) quantification of T₁ and T₂ signals measured in c) expressed as the relative contrast enhancement (RCE).

### Examples

### Materials and Methods

### Example 1: Synthesis of the NPs of the present invention

In a 0.5 - 2 mL MW vial with a suitable magnetic stirrer, 490 µL of milli Q water, 150 µL of a 50 mg/mL solution of iron (III) chloride hexahydrate and 160 µL of a 50 mg/ml sodium citrate were added and then 100 µL of ²²³RaCl₂ solution and hydrazine hydrate were added to the vial. The vial was closed with a septum cap and stirred under MW at 100°C for 10 min.

After this time the reaction was cooled down with pressurized air to 50°C and was purified with a size exclusion Sephadex-G25 prepacked column.

### Example 2: Characterization of the NPs obtained in Example 1

Iron oxide nanoparticles have a core size of 4 ± 2 nm and a hydrodynamic size (core plus organic coating) of 15 ± 5 nm, thus a layer of organic material (sodium citrate in this case) of around 11 nm.

Nanoparticles core-doped with radioisotope ²²³Ra with a radiolabelling yield (RLY) of 85 ± 1 % (this RLY refers to the amount of ²²³Ra incorporated in the core referred to the amount initially used). Radiochemical yield (RLY) was measured as the percentage resultant of dividing the purified sample activity between the total activity (sample activity + purification column activity).

### Example 3: Study of the stability in serum of the NPs obtained in Example 1

One of the major problems when using ²²³Ra is the stability of the labelling when exposed to human (or animal) serum. Until now, there are not many options to have ²²³Ra labelled and stable *in vivo.* For this reason, the only approved compound for humans is ²²³RaCl₂. Then, the stability *in vivo* values are key.

*In vitro* stability of ²²³Ra-IONP of the present invention was tested in human serum. For this purpose, 150 µL of radiolabelled nanoparticles of the present invention were incubated with 150 µL of human serum (Sigma Aldrich) at 37°C. The stability was evaluated at 1, 24, 48 and 120 h measuring the serum stability as the percentage of activity that remains in the filter after three steps of ultrafiltration with AMICON 30kDa with PBS 1X as solvent for re-suspending the sample between filtrations.

The results are shown in Fig 2, which clearly shows that the stability of the NPs remains even after 120 h.

### Example 4: Preclinical MRI studies of glioma model

The inventors used a glioblastoma mouse model by injecting glioma C6 cells into NOD-SCID mice. They intravenously injected 0.06 mmol Fe/Kg and imaged the brain 90 min post-injection. The results show different images for the developed glioblastoma in the brain; baseline images were recorded, and after the injection of the nanoparticles, the T₁ signal was measured (Figure 3). An increase in RCE of tumor relative to contralateral-healthy brain was observed in T₁W images 90 minutes after nanoparticle injection (44.30 ± 4.56 %) compared to the pre-injection RCE (0.93 ± 0.97 %). To further demonstrate the presence of nanoparticles in the lesion, we performed T₁ and T₂ imaging. The results are shown in figure 3c, clearly showing the positive signal in the tumor and how the same areas turned black owing to the negative contrast that the nanoparticles were able to provide. These changes were measured using RCE (Figure 3b). The results confirm that the images show a clear increase in the brightness of the signal in T₁, which reduces when switching to T₂-weighted imaging. T₂W images acquired at 90 minutes after nanoparticle injection showed a decrease in RCE of tumor relative to contralateral (-49.67 ± 15.49 %) compared to the pre-injection RCE (12.68 ± 4.54 %).

### In vivo glioma model

Authenticated glioma C6 cells from the American Type Culture Collection (number CCL-107, Manassas, VA, USA) were cultured with DMEM supplemented with 10% fetal bovine serum, penicillin gentamicin and streptomycin. Cells were maintained in an incubator at 37 °C and 5 % CO₂ until reaching confluence, when were detached and counted to generate the glioma model.

The orthotopic glioma model was generated in three NOD-SCID mice of 8-10 weeks of age (as previously reported in *Pharmaceutics* 2021, 13(8), 1258). Briefly, animals Mice were then placed in a stereotaxic device where anesthesia was maintained through a nose mask (1-1.5% isoflurane/O₂) and animal's eyes were covered with Vaseline to prevent them from drying out. Then, a midline incision on the skull was made using a scalpel and a burr hole was performed 0.23 mm right to the Bregma using a 25G needle. Then, 105 C6 cells in 10 µL of DMEM with 30% Matrigel were injected to a depth of 0.33 mm in the right caudate nucleus with a Hamilton syringe. After 5 minutes, the syringe was carefully removed, the hole sealed with bone wax and the scalp sutured. Animals were injected subcutaneously with buprenorphine as analgesia 30 minutes before the procedure and the following 2 days (0.1 mg/kg).

### Preclinical MRI studies of glioma model

MRI studies were performed on a horizontal 7.0-Tesla Bruker Biospec^{®} system (Bruker Medical Gmbh, Ettlingen, Germany) using a 40 mm transmitter coil with a 23 mm mouse brain surface coil as a receiver. MR images were acquired using ParaVision 6.0.1 software operating on a Linux environment. Animals were anesthetized as described in the tumor generation protocol and anesthesia was also maintained during the whole MRI experiment through a nose mask. Body temperature was maintained at 37°C using a heated waterbed and respiratory rate was also monitored during the MRI study.

Tumor development was followed-up by T₂-weighted MRI twice a week. Three weeks after tumor generation, mice were subjected to the nanoparticle-experiment. First, a baseline study was performed prior to nanoparticles injection acquiring two types of MR images in an axial orientation with 14 slices with 1 mm of slice thickness, a matrix size of 256 x 256 s and a field of view (FOV)= 23 x 23 mm2 corresponding to an in-plane resolution of 90 x 90 µm²:
-T₂ weighted (T₂W) image using a rapid acquisition with relaxation enhancement (RARE) sequence, with a repetition time (TR)= 2500 ms, effective echo time (TE eff) = 26 ms, RARE factor= 8, number of averages (Av)= 4, total acquisition time (TAT)= 4 min.

-T₁ weighted (T₁W) image using a multi-slice multi-echo sequence sequence (MSME), with a TR= 300 ms, echo time TE eff= 10 ms, Av= 3, TAT= 3 min, 50 s.

Then, mice were injected intravenously with 200 µl of the nanoparticle preparation and 90 minutes after the injection, animals were again studied by MRI using the same set of images acquired in the baseline study.

Images were analyzed using imaged (National Institute of Health, NIH) by manually selecting two regions of interests (ROIs) with a size of approximately 20 pixels in a representative slice. One ROI was selected within the tumor region with active contrast uptake and the other in the contralateral-healthy brain. The percentage of relative contrast enhancement (RCE) of the signal intensity (SI) of the tumor ROI vs SI of the contralateral ROI was calculated at baseline (pre-nanoparticle injection) and in the 90 min post-injection MRI studies for T1W and T2W images, respectively.

## Claims

1. Nanoparticles **characterized in that** they comprise:
an iron oxide core doped with radioisotope ²²³Ra and
a coating layer of an organic compound selected from: sodium citrate, dextran, carboxydextran, poly(vinyl alcohol) (PVA), 2,3-dimercaptosuccinic acid, 1-aminoglucose and glutamate.

2. Nanoparticles, according to claim 1, wherein the iron oxide is selected from FeO, Fe₃O₄, Fe₄O₅, Fe₂O₃ or FeOs.

3. Nanoparticles, according to claim 1 or 2, wherein the average diameter of the iron oxide core is between 2 and 6 nm, as measured by Transmission Electron Microscopy (TEM), and/or the hydrodynamic average diameter of the nanoparticles is between 10 and 20 nm, as measured by dynamic light scattering measurement (DLS).

4. Nanoparticles, according to any of previous claims, wherein the organic compound is sodium citrate.

5. Method for preparing the nanoparticles described in any of previous claims, said method comprises the following steps:
a) preparing a solution in water of a combination of: an iron salt, a ²²³Ra salt, hydrazine hydrate, and an organic compound selected from sodium citrate, dextran, carboxydextran, poly(vinyl alcohol) (PVA), 2,3-dimercaptosuccinic acid, 1-aminoglucose and glutamate.
b) subjecting the mixture obtained in step (a) to a temperature between 90 and 120°C with microwave (MW) irradiation at 220-300W for 3-11 min.

6. The method, according to claim 5, wherein the iron salt is selected from the group consisting of FeCl₃, Fe(SO₄), Fe₂(SC₄)₃, Fe(NO₃)₃, FeCOs, Fe(OH)₂, Fe(OH)₃ and any hydrate thereof..

7. The method, according to any of previous claims 5 or 6, wherein the iron salt is iron salt is iron (III) chloride hexahydrate.

8. The method, according to any of previous claims 5 to 7, wherein the ²²³Ra salt is ²²³RaCl₂.

9. The method, according to any of previous claims 5 to 8, wherein the organic compound is sodium citrate.

10. The method, according to any of previous claims 5 to 9, wherein step b) is carried out at 100°C for 10 min with MW irradiation.

11. The method, according to any of previous claims 5 to 10, wherein the nanoparticles obtained in step b) are purified by size-exclusion chromatography (SEC).

12. Pharmaceutical composition comprising the nanoparticles described in anyone of claims 1 to 4 and at least one pharmaceutically acceptable vehicle or excipient.

13. The nanoparticles described in anyone of claims 1 to 4, or a composition described in claim 12, for use as a medicament.

14. The nanoparticles described in anyone of claims 1 to 4, or a composition described in claim 12, for use as radiotheranostic agents.

15. The nanoparticles described in anyone of claims 1 to 4, or a composition described in claim 12, for use in the treatment of cancer.
